# EUROPEAN PATENT APPLICATION

(11) **EP 4 249 547 A1**
(43) Date of publication of application: **27.09.2023**
(21) Application number: 21893157.4
(22) Date of filing: 16.11.2021
(51) Int. Cl.: C08J 11/10, C08J 11/24

(54) **PROCESS FOR THE DEPOLYMERIZATION OF POLYETHYLENE TEREPHTHALATE**

(30) Priority: 17.11.2020 BR 102020023482
(71) Applicant: Petroleo Brasileiro S.A. - PETROBRAS, 20031912 Rio de Janeiro (BR); Universidade Federal Do Rio De Janeiro - UFRJ, 21941-901 Rio de Janeiro (BR)
(72) Inventor: MACHADO DE CASTRO, Aline, 20771-002 Rio de Janeiro (BR); CARNIEL DE OLIVEIRA, Adriano, 22631-450 Rio de Janeiro (BR); NICOMEDES JUNIOR, José, 24210-455 Niterói (BR); O. M. A. DE SOUZA, Rodrigo, 22795-315 Rio de Janeiro (BR)
(74) Representative: Clarke, Modet y Cía., S.L.
(86) International application number: PCT/BR2021/050496
(87) International publication number: WO 2022/104442

(57) **Abstract**

The present invention relates to a process for the depolymerization of resistant polyethylene terephthalate (PET) by combining pretreatment steps for the purpose of decreasing the recalcitrance of PET from cables used in the oil and gas exploration and production (E&P) sector, which steps improve the performance of the enzymes used in the step of enzymatic depolymerization. The invention is used mainly for managing solid waste generated from E&P activity, whereby the products obtained can benefit this industrial sector in a circular manner, or else be used in the petrochemical sector, while at the same time contributing towards protecting the environment when operating and decommissioning off-shore units.

## Description

### Field of the Invention

The present invention relates a process for the depolymerization of resistant polyethylene terephthalate (PET) with application in the area of solid waste management that combines steps to reduce the recalcitrance of PET from cables used in the oil and gas exploration and production (E&P) area, which improve the performance of the enzymes used in the step of enzymatic depolymerization.

### Description of the State of the Art

For decades, polyethylene terephthalate (PET) fibers have been replacing steel as the main component of mooring cables for floating marine units, due to their high mechanical strength and high durability. In addition to mooring platforms, these cables are widely used in several other routine operations in the off-shore oil and gas industry, such as moving rigs, pull-backs, torpedoes launching, towing, etc.

Currently, the waste generated after the use of PET cables is accumulated in yards that occupy large areas and are subsequently auctioned at very low prices. With the prospect of an increase in the volume of generated material and the increasing demand from regulatory environmental agencies regarding the proper disposal of waste after the decommissioning of maritime units, the need for the development of alternative methods for recycling the fibers that make up the mooring systems also grows. Ideally, also adding value to products.

In this context, an ideal process should allow the complete depolymerization of PET, preserving the physicochemical characteristics of its components and generating drop-in monomers for new PET synthesis processes. It is known that this objective cannot be achieved through mechanical recycling and, therefore, a biotechnological approach is proposed.

Within the scope of biotechnological processes, the literature shows some examples of the use of enzymes for degradation and recovery of PET oligomers and monomers. Enzymes can catalyze the hydrolysis of ester bonds in polyesters or act as catalysts for the oxidation of functional groups, making synthetic polymers more hydrophilic. Its performance also extends to the alteration of characteristics such as crystallinity, orientation and morphology of polymers by the same mechanism of hydrolysis or oxidation reactions, which can occur in the central or side chain of polymers, parameters that influence their degradation. However, some polymers are less susceptible than others to enzymatic catalysis as described in SHAH, A. A. et at., "Biological degradation of plastics: a comprehensive review", Biotechnology Advances, volume 26, pages 246 to 265, 2008.

However, most articles focused on the enzymatic degradation of polymers study the enzymatic degradation in less recalcitrant polymers, that is, with a lower degree of crystallinity and/or lower molar mass and/or lower degree of structural organization. Document BR 1020140205098 A2 indicates good degrees of depolymerization of PET from packaging (bottles, food trays, etc.), which are less recalcitrant. The same approach, however, is not effective on PET from cables used in E&P activities, since this type of material is developed to have high durability and very resistant properties, as described in LIU, H. et at. "An experimental investigation on nonlinear behaviors of synthetic fiber ropes for deepwater moorings under cyclic loading", Applied Ocean Research, volume 45, pages 22 to 32.

Thus, there is a growing concern to provide a more appropriate treatment for this waste, which has the potential to cause environmental problems. Alternatives that allow the recycling and reuse of this material can bring advantages both for the companies that manufacture the polymer and for those that use it.

Document BR 1020160155223 A2 discloses an enzymatic process of depolymerization of PET, in which there is the use of enzymes such as cutinase or lipase. In addition, it mentions a pretreatment step with ethylene glycol, however this step is optional and is aimed at polymers with a low degree of crystallinity, that is, with low recalcitrance.

Patent US 10124512 B2 relates to a biological method to depolymerize at least one polymer of a plastic product and recover the resulting monomers, which can be further reprocessed to synthesize new polymers and manufacture new plastic products. However, such patent does not propose a pretreatment process for depolymerization of resistant PET, that is, with a high degree of crystallization, in seawater.

The document by LACERDA, C.E. O. "Reciclagem química do poli(tereftalato de etileno) - PET pós-consumo via hidrólise", Thesis (Doctorate), Federal University of Pernambuco, 2019, aims to investigate important variables in the process of chemical recycling of post-consumption PET in the form of flakes through hydrolysis, using NaOH, KOH, H₂SO₄ and HNO₃. Despite referring to the depolymerization of PET and the petrochemical industry, it does not propose a depolymerization procedure that uses enzymes such as lipase or cutinase, it does not contemplate the use of pretreatments, with the aim of reducing recalcitrance (by reducing crystallinity) of more resistant PET and much less mentions a depolymerization procedure in sea water.

Thus, no prior art document discloses a process for depolymerizing resistant PET such as that of the present invention.

In order to solve such problems, the present invention was developed, through which it contemplates the use of pretreatments, aiming to reduce the recalcitrance mainly through the reduction of the crystallinity of the PET from cables used in E&P, thus improving the performance of the enzymes used in the enzymatic depolymerization step. The types of pretreatment employed proved to be effective in improving the yield of the process as a whole. In addition, it can serve E&P decommissioning projects by conducting the depolymerization of cables in sea conditions, or the proper disposal of these materials taken ashore. The present invention adds value to the unserviceable cables of E&P activities, contributing to the monetization of the process chain.

The processes of the present invention are operationally safe, as they do not use toxic solvents and operate at atmospheric pressure. Furthermore, obtaining PET degradation products for a new polymer synthesis potentially reduces the consumption of new amounts of monomers (terephthalic acid and ethylene glycol), which largely come from fossil origin.

### Brief Description of the Invention

The present invention relates to a process for the depolymerization of resistant polyethylene terephthalate (PET) in order to reduce recalcitrance mainly by reducing the crystallinity of PET from cables used in E&P, thus improving the performance of the enzymes used in the enzymatic depolymerization step.

The application of the invention is mainly in the management of solid waste generated in E&P activities, and the products obtained can circularly benefit this industrial sector, or even be used in the petrochemical area.

### Brief Description of the Drawings

The present invention will be described in more detail below, with reference to the attached figures which, in a schematic way and not limiting the inventive scope, represent examples of its realization. In the drawings:
- Figure 1 illustrates a scheme of the process for depolymerization of resistant PET of the present invention, where (1) stream of PET, (2) stream of solvent, (3) pretreatment reactor, (4) stream of enzyme, (5) stream of reaction solvent, which may be aqueous or organic medium, (6) reactor of the enzymatic depolymerization process and (7) stream of depolymerization products (oligomers and monomers), are represented;
- Figure 2 illustrates time courses of release of terephthalic acid (TPA) monomers (squares) and mono(hydroxyethyl terephthalate) (MHET) (triangles), in reactions without pretreatment (B), and with ultrasound (US) and microwave (MO) pretreatment, using buffer solution as solvent in the pretreatment and depolymerization steps;
- Figure 3 illustrates time courses of release of TPA (squares) and MHET (triangles) monomers in reactions without pretreatment (B), and with ultrasound (US) and microwave (MO) pretreatment, using ethylene glycol as solvent in the pretreatment step and buffer solution in the depolymerization step;
- Figure 4 illustrates time courses of TPA monomer release, in reactions without pretreatment (blank), and with pretreatment with chemical surfactants or enzymes;
- Figure 5 illustrates time courses of release of TPA and MHET monomers in the depolymerization reactions of cable PET in seawater. Blank assays (B) without enzyme, and assays with enzyme (E).

### Detailed Description of the Invention

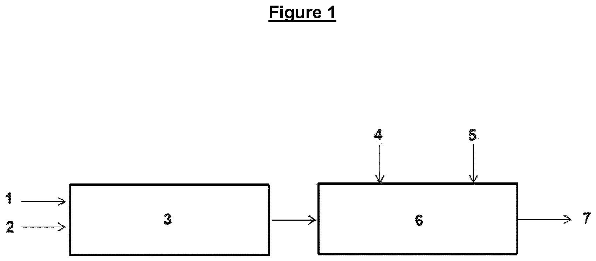

The process for depolymerizing resistant polyethylene terephthalate (PET) according to the present invention and illustrated in Figure 1 comprises two steps, the pretreatment step and the enzymatic depolymerization step, in which the pretreatment step can be chosen from:

### 1. Microwave pretreatment:

In a microwave reactor, adding the solvent and the PET, and conducting the pretreatment for 5 minutes to 4 hours, at a temperature of 30 °C to 250 °C, and stirring from 20 to 800 rpm. The solvent can be a buffer solution, aqueous solution with additive, or ethylene glycol, and the PET/solvent ratio can be from 1 g/3 mL to 1 g/50 mL.

After the pretreatment, adding a buffer solution and the enzyme, and conducting the enzymatic depolymerization reaction in a second reactor for 6 hours to 60 days at a temperature of 20 °C to 80 °C. The PET/buffer ratio can be from 1 g/3 mL to 1 g/50 mL and the amount of enzyme can be from 0.001 to 0.5 g protein/g PET. At the end of depolymerization, recovering the solvent monomers and oligomers for reuse.

### 2. Pretreatment with ultrasound:

In an ultrasound reactor, adding the solvent and the PET, and conducting the pretreatment for 3 minutes to 4 hours at a temperature of 15 °C to 80 °C. The solvent can be buffer solution, aqueous solution with additive, or ethylene glycol, and the PET/solvent ratio can be from 1 g/ 3 mL to 1 g/50 mL.

After the pretreatment, adding a buffer solution and the enzyme, and conducting the enzymatic depolymerization reaction in a second reactor for 6 hours to 60 days at a temperature of 20 °C to 80 °C. The PET/buffer ratio can be from 1 g/3 mL to 1 g/50 mL and the amount of enzyme can be from 0.001 to 0.5 g protein/g PET. At the end of depolymerization, recovering the solvent monomers and oligomers for reuse.

### 3. Pretreatment with surfactants:

In a continuous stirred tank reactor (CSTR), adding buffer solution or aqueous solution with additive, and PET, and conducting the pretreatment for 10 seconds to 24 hours at a temperature of 20 °C to 80 °C. The PET/solvent ratio can be from 1 g/3 mL to 1 g/50 mL.

After the pretreatment, adding the depolymerization enzyme, and conducting the enzymatic depolymerization reaction in the same reactor for 6 hours to 60 days at a temperature of 20 °C to 80 °C. The amount of enzyme can be from 0.001 to 0.5 g protein/g PET. At the end of the depolymerization, recovering the solvent monomers and oligomers for reuse.

### 4. Depolymerization in sea water:

In a continuous stirred tank reactor (CSTR), adding seawater, enzyme and PET, and conducting the enzymatic depolymerization reaction, in the same reactor, for 6 hours to 60 days, at a temperature of 10 °C to 50 °C. The amount of enzyme can be from 0.001 to 0.5 g protein/g PET. The PET/seawater ratio can be from 1 g/3 mL to 1 g/50 mL. At the end of the depolymerization, recovering the solvent monomers and oligomers for reuse. The reaction can also be conducted in a wave bioreactor, which can be installed on floating units at sea, using the movement of sea waves to mix the reaction environment, which maintains the good performance of the enzyme. In this case, the reaction temperature can extend up to 80 °C and the reaction time can extend up to 120 days. At the end of the depolymerization, recovering the solvent monomers and oligomers for reuse.

The approaches described here can also be applied to other sources of PET, such as packaging.

The buffer solution used in the present invention can be chosen from sodium phosphate, potassium phosphate or tris-HCl. Unbuffered aqueous solution can also be used, with pH control of the solution through the addition of an alkali solution (such as NaOH or KOH).

In the pretreatment with surfactants, liquid surfactants, such as tween 20, tween 80 or an enzyme preparation based on cellulases, or solid surfactants, such as PEG 4000 and bovine serum albumin, can be selected.

The biocatalyst used in the present invention is lipase, cutinase, esterase or PETase type enzymes.

All of these processes described here can be used in sequence with other types of PET recycling processes, such as chemical and physical processes.

### EXAMPLES:

In the examples below, examples are given in order to illustrate some particular embodiments of the present invention and should not be interpreted as limiting the same.

In all examples, PET samples from platform anchoring cables, used for 15 years, were used.

### EXAMPLE 1: Pretreatment with buffer solution

200 mg of PET cable filaments were placed in contact with 3 mL of sodium phosphate buffer solution 200 mM pH 7, in a microwave reactor, for 1 hour at 70 °C, or in an ultrasound reactor, at 22 °C for 1 hour. To the resulting suspension, another 7 mL of the same buffer solution, lipase/cutinase type enzyme at a concentration of 0.01 g protein/g PET, was added and allowed to react at 60 °C for up to 14 days. 0.2% w/v sodium azide solution was added to prevent microbial growth and monomer consumption. Blank tests were also performed, adding 10 mL of buffer solution and 0.01 g protein/g PET directly to 200 mg of PET cable, without adding the enzyme. The other depolymerization conditions of the blank tests were the same used for the samples.

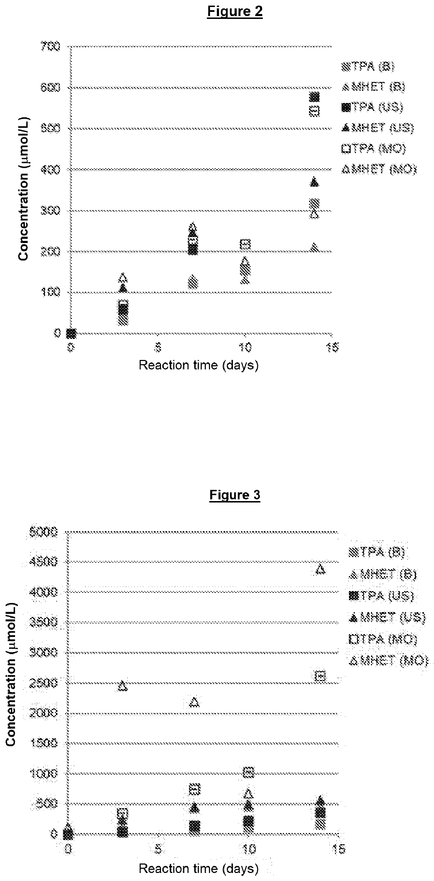

The concentrations of terephthalic acid (TPA) and mono(hydroxyethyl terephthalate) (MHET) products during the depolymerization were determined by high performance liquid chromatography, as described by CASTRO, A. M. et al. "Screening of commercial enzymes for polyethylene terephthalate (PET) hydrolysis and synergy studies on different substrate sources", Journal of Industrial Microbiology and Biotechnology, volume 44, pages 835 to 844, 2017, and are shown in Figure 2.

### EXAMPLE 2: Pretreatment with ethylene glycol

200 mg of PET cable filaments were placed in contact with 3 ml of ethylene glycol, in a microwave reactor, for 1 hour at 70 °C, or in an ultrasound reactor, at 22 °C for 1 hour. To the resulting suspension, 7 mL of sodium phosphate buffer solution 200 mM pH 7, lipase/cutinase type enzyme at a concentration of 0.01 g protein/g PET, were added and left to react at 60 °C for up to 14 days. 0.2% w/v sodium azide solution was added to prevent microbial growth and monomer consumption. Blank tests were also performed, adding 10 mL of buffer solution and 0.01 g protein/g PET directly to 200 mg of PET cable, without adding the enzyme. The other depolymerization conditions of the blank tests were the same used for the samples.

The concentrations of terephthalic acid (TPA) and mono(hydroxyethyl terephthalate) (MHET) products during the depolymerization were determined by high performance liquid chromatography, as described by CASTRO, A. M. et al. "Screening of commercial enzymes for polyethylene terephthalate (PET) hydrolysis and synergy studies on different substrate sources", Journal of Industrial Microbiology and Biotechnology, volume 44, pages 835 to 844, 2017, and are shown in Figure 3.

### EXAMPLE 3: Pretreatment with surfactants

100 mg of PET cable filaments were placed in contact with 10 mL of Tris-HCl buffer solution 397 mM pH 8,95 and different surfactants, in the proportion of 0.10% v/v, for liquid surfactants (Tween 20, tween 80 or enzymatic preparation based on cellulases) or 1:1 w/w for solid surfactants (PEG 4000 and bovine serum albumin - BSA). The suspension was vigorously stirred on a vortex-type device for about 30 seconds. Then, the depolymerization enzyme, lipase/cutinase, was added at a concentration of 0.06 g protein/g PET and allowed to react at 67 °C under agitation at 25 rpm, for up to 14 days. 0.2% w/v sodium azide solution was added to prevent microbial growth and monomer consumption. Blank tests were also performed, adding 10 mL of buffer solution and 0.06 g protein/g PET directly to 100 mg of PET cable, without adding the enzyme. The other depolymerization conditions of the blank tests were the same used for the samples.

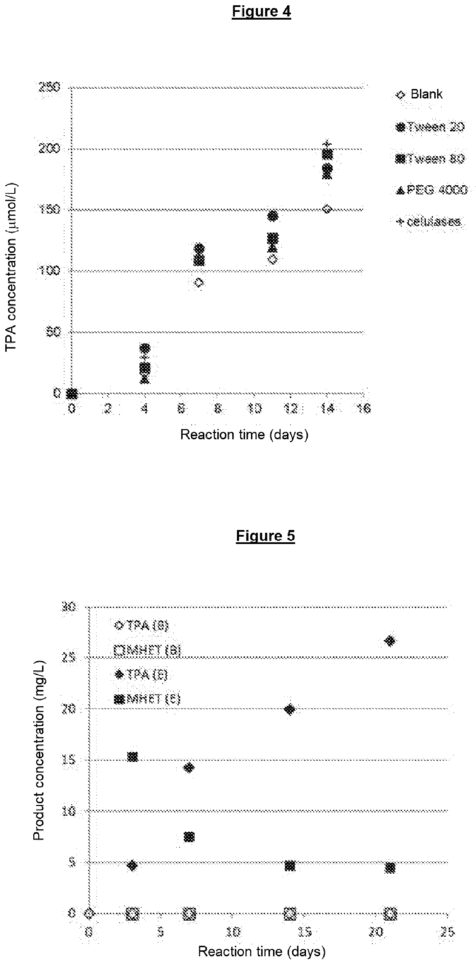

The concentrations of terephthalic acid (TPA) and mono(hydroxyethyl terephthalate) (MHET) products during the depolymerization were determined by high performance liquid chromatography, as described by CASTRO, A. M. et al. "Screening of commercial enzymes for polyethylene terephthalate (PET) hydrolysis and synergy studies on different substrate sources", Journal of Industrial Microbiology and Biotechnology, volume 44, pages 835 to 844, 2017, and are shown in Figure 4.

### EXAMPLE 4: Depolymerization in sea water

350 mg of PET cable filaments were placed in contact with 10 mL of natural sea water. Then, the depolymerization enzyme, lipase/cutinase, was added at a concentration of 0.06 g protein/g PET and allowed to react at 26 °C under orbital stirring at 100 rpm, for up to 21 days. 0.2% w/v sodium azide solution was added to prevent microbial growth and monomer consumption. Blank tests were also performed, adding 10 mL of buffer solution directly to 350 mg of PET cable, without adding the enzyme. The other depolymerization conditions of the blank tests were the same used for the samples.

The concentrations of terephthalic acid (TPA) and mono(hydroxyethyl terephthalate) (MHET) products during the depolymerization were determined by high performance liquid chromatography, as described by CASTRO, A. M. et al. "Screening of commercial enzymes for polyethylene terephthalate (PET) hydrolysis and synergy studies on different substrate sources", Journal of Industrial Microbiology and Biotechnology, volume 44, pages 835 to 844, 2017, and are shown in Figure 5.

It should be noted that, although the present invention has been described in relation to the attached drawings, it may undergo modifications and adaptations by persons skilled in the art, depending on the specific situation, but provided that it is within the inventive scope defined herein.

## Claims

1. PROCESS FOR THE DEPOLYMERIZATION OF POLYETHYLENE TEREPHTHALATE, **characterized by** comprising the following steps:
a) adding polyethylene terephthalate and a solvent in a first reactor to carry out the pretreatment step;
b) then, adding to the suspension resulting from step (a) an aqueous solution and an enzyme to carry out the enzymatic depolymerization step in a second reactor for 6 hours to 60 days and at a temperature between 20 °C to 80 °C;
c) recovering the solvent monomers and oligomers for their reuse.

2. PROCESS, according to claim 1, **characterized in that** the solvent is ethylene glycol or a buffer solution.

3. PROCESS, according to any one of claims 1 or 2, **characterized in that** the aqueous solution is sodium phosphate buffer, potassium phosphate or tris-HCl, or aqueous solution added with alkaline solution, for pH control.

4. PROCESS, according to claim 1, **characterized in that** the pretreatment step is carried out with a microwave reactor, ultrasound reactor or with the addition of surfactants or enzymes.

5. PROCESS, according to claim 4, **characterized in that** the pretreatment is conducted in a microwave reactor for 5 minutes to 4 hours, at a temperature between 30 °C to 250 °C and agitation between 20 to 800 rpm.

6. PROCESS, according to claim 4, **characterized in that** the pretreatment is conducted in an ultrasound reactor for 3 minutes to 4 hours and at a temperature between 15 °C and 80 °C.

7. PROCESS, according to claim 4, **characterized in that** the pretreatment with surfactants or enzymes is conducted in a continuous stirred tank reactor (CSTR) for 10 seconds to 24 hours and at a temperature between 20 °C to 80 °C.

8. PROCESS, according to any one of claims 1 or 7, **characterized in that** the pretreatment with surfactants and the depolymerization are conducted in the same reactor.

9. PROCESS, according to claim 7, **characterized in that** the surfactants are liquid or solid.

10. PROCESS, according to claim 9, **characterized in that** the liquid surfactants are tween 20, tween 80 or enzymatic preparation based on cellulases.

11. PROCESS, according to claim 9, **characterized in that** the solid surfactants are PEG 4000 or serum bovine albumin.

12. PROCESS, according to claim 1, **characterized in that** the ratio of PET/solvent is between 1 g/3 mL to 1 g/ 50 mL.

13. PROCESS, according to claim 1, **characterized in that** the amount of enzyme is between 0.001 to 0.5 g protein/ g PET.

14. PROCESS, according to claim 1, **characterized in that** the enzyme is of lipase, cutinase, esterase or PETase type.

15. PROCESS, according to claim 1, **characterized in that** only the depolymerization step occurs in the same continuous stirred tank reactor (CSTR), adding sea water, enzyme and PET, for 6 hours to 60 days and at a temperature between 10 °C to 50 °C.

16. PROCESS, according to claim 1, **characterized in that** only the depolymerization step occurs in a wave bioreactor, which can be installed in floating units at sea, using sea water wave motion for mixing the reaction environment, and sunlight for heating the mixture, adding sea water, enzyme and PET, for 6 hours to 120 days and at a temperature between 10 °C to 80 °C.
